# EUROPÄISCHE PATENTANMELDUNG

(11) **EP 0 957 441 A2**
(43) Veröffentlichungstag der Anmeldung: **17.11.1999**
(21) Anmeldenummer: 99108841.0
(22) Anmeldetag: 04.05.1999
(51) Int. Cl.: G06F 19/00

(54) **Verfahren und Vorrichtung zur komprimierten optischen Darstellung medizinischer Daten**

(30) Priorität: 14.05.1998 DE 19821761
(71) Anmelder: B. BRAUN MELSUNGEN AG, 34212 Melsungen (DE)
(72) Erfinder: Schmid, Günter, 82110 Germering (DE); Becker, Uwe, 82194 Gröbenzell (DE); Herzner, Josef, 81541 München (DE)
(74) Vertreter: Selting, Günther, Dipl.-Ing.

(57) **Zusammenfassung**

Die Erfindung betrifft Verfahren zur gleichzeitigen komprimierten optischen Darstellung der Daten dreier oder mehrerer medizinisch relevanten Parameter, bei dem die Daten der drei oder mehreren medizinisch relevanten Parameter in einem drei- oder mehrdimensionalen Koordinatensystem dargestellt werden, bei dem das drei- oder mehrdimensionale Koordinatensystem zusammen mit den darin dargestellten Daten der medizinisch relevanten Parameter mit Hilfe einer vorgegebenen Rechenvorschrift in ein zweidimensionales Koordinatensystem transformiert wird und bei dem das Ergebnis dieser Transformation auf einer Anzeigefläche eines Anzeigemittels angezeigt wird, wobei die Daten mindestens eines der medizinisch relevanten Parameter einen in seinen wesentlichen Merkmalen zeitlich periodischen Verlauf aufweisen. Des weiteren betrifft die Erfindung eine Anzeigevorrichtung, bei der das erfindungsgemäße Verfahren realisiert ist.

## Beschreibung

Die vorliegende Erfindung bezieht sich auf medizinische Systeme und insbesondere auf Patientenüberwachungssysteme zur Erfassung, Verarbeitung und Anzeige von Daten kritisch kranker Patienten.

In der Überwachung kritisch kranker Patienten ist es häufig notwendig, eine Vielfalt medizinischer Daten über den Zustand eines Patienten zu gewinnen. Hierfür stehen eine Reihe von Möglichkeiten zur Verfügung. Deren häufigste sind die Verwendung von Patientenmonitoren zur Erfassung physiologischer Parameter, wie des Elektrokardiogramms, des Blutdrucks, der Sauerstoffsättigung oder der Atmung, die Laboranalyse physiologischer Proben, wie z.B. Blutproben oder Gewebeproben, und, bei beatmeten Patienten, die Auswertung von Einstellungen und Meßwerten des Beatmungsgeräts. Insbesondere die von einem Patientenüberwachungsmonitor erfaßten Daten sind in der jüngeren Vergangenheit in ihrer Quantität stark gestiegen, da sich einerseits der technische Fortschritt auf dem Gebiet der Sensortechnik und der Mikroelektronik hier nutzbringend auswirkt, und andererseits das steigende Verständnis für die medizinischen Wirkungszusammenhänge die Forderung nach Erfassung weiterer Parameter vorantreibt.

Diese Entwicklung hat zu dem Zustand geführt, daß die Zahl der physiologischen Parameter, die von einem Patienten in modernen Intensivstationen und Operationsräumen erfaßt, verarbeitet und angezeigt werden kann so hoch ist, daß selbst sehr gut gestaltete Anzeigemittel kaum mehr eine schnelle Erfassung und sinnvolle Auswertung der Datenflut durch das medizinische Fachpersonal erlauben. Da der Mensch nur in der Lage ist, eine relativ begrenzte Anzahl von Kurven und sich ändernden Meßwerten simultan im Auge zu behalten und korrekte Schlußfolgerungen aus diesen Beobachtungen zu ziehen, zeigt sich bei Patientenüberwachungssystemen nach dem derzeitigen Stand der Technik deutlich der Effekt des sogenannten "mental overload" also der Überlastung der geistigen Aufnahmefähigkeit. Es ergibt sich somit die Notwendigkeit, Verfahren zur Vermeidung dieses genannten Effekts zu suchen.

Daß es einen deutlichen Zusammenhang zwischen der Form der Darstellung physiologischer Daten und der Fähigkeit zu deren Interpretation durch das medizinische Fachpersonal gibt, wurde beispielsweise in einer in Anesthesiology Vol.. 83, S. 1184-1193 veröffentlichten Studie mit dem Titel "Visual Display Format Affects the Ability of Anesthesiologists to Detect Acute Physiologic Changes" gezeigt. In dieser Studie wurden unterschiedliche Präsentationsformen physiologischer Daten und die Schnelligkeit und Richtigkeit, mit der Veränderungen dieser physiologischen Daten vom medizinischen Fachpersonal erkannt werden, untersucht. Ein Ergebnis ist die deutlich schnellere und richtigere Interpretation der physiologischen Daten, wenn deren Präsentation in graphischer Form erfolgt. Zu einem ähnlichen Ergebnis kommt die im Journal of Clinical Monitoring Vol. 13, S. 249-259 erschienene Untersuchung "An Integrated Graphic Data Display Improves Detection and Identification of Critical Events During Anesthesia", bei der eine graphischen Anzeige von 30 anästhesiebezogenen Parametern mit einer bei Patientenmonitoren nach gegenwärtigem Stand der Technik üblichen Darstellungweise mit Hilfe von Kurven und Zahlenwerten verglichen wird. Dabei zeigte die konventionelle Kurven- und Zahlendarstellung zeitliche Nachteile von mehreren Minuten gegenüber einer neuen graphischen Anzeige im Erkennen relevanter Ereignisse durch das medizinische Fachpersonal.

Da, wie oben beschrieben, die Zahl der an einem kritisch kranken Patienten überwachten Parameter immer mehr ansteigt, sich andererseits jedoch die Größe der Überwachungsgeräte kaum ändert oder sich infolge des technischen Fortschritts sogar verringert, ergibt sich ein Dilemma bei der Anzeige all dieser Parameter auf der Anzeige eines Überwachungsgeräts. Häufig werden bestimmte überwachte Parameter nicht oder nicht in ausreichender Größe und Qualität auf der Anzeige eines Überwachungsgeräts angezeigt, da der zur Verfügung stehende Platz auf der Anzeige nicht für eine qualitativ befriedigende Anzeige aller überwachten Parameter ausreicht. Andere Parameter, deren Überwachung zwar medizinisch vorteilhaft wäre, werden nicht überwacht, da die Anzeigemöglichkeiten eines Überwachungsgeräts bereits durch andere, medizinisch wichtigere-Parameter ausgeschöpft sind.

Der vorliegenden Erfindung liegt daher die Aufgabe zugrunde, ein Verfahren und eine Vorrichtung zur optischen Darstellung von mittels medizinischen Systemen erfaßten medizinischen Daten zu schaffen, wobei eine rasche Erfaßbarkeit und Auswertung der Daten durch einen Betrachter gewährleistet werden kann.

Die vorliegende Erfindung vermeidet die Nachteile der Anzeige medizinischer Daten, wie sie in bisherigen medizinischen Systemen üblich ist, durch eine deutliche Verringerung des Platzbedarfs für die Anzeige dieser medizinischen Daten. Darüber hinaus ist eine Anzeige, welche gemäß der durch die vorliegende Erfindung beschiebenen Methode gestaltet ist, in ihrer physiologischen Bedeutung vom medizinischen Fachpersonal deutlich schneller und leichter zu erfassen, als Anzeigen gemäß dem bisherigen Stand der Technik.

Es wird von mindestens drei medizinisch relevanten Daten ausgegangen, die in der Regel von Meßwerterfassungsvorrichtungen gewonnene Meßwerte umfassen oder von solchen Meßwerten abgeleitet werden können. Es wird unterschieden zwischen sich relativ schnell ändernden Daten, wie z.B. dem Elektrokardiogramm, dem Elektroenzephalogramm, der arteriellen Pulskurve, der Kurve des Atemwegsdrucks, etc, und sich relativ langsam ändernden Daten, wie z.B. der Herzfrequenz, der Werte für systolischen und diastolischen Blutdruck, des Wertes der Sauerstoffsättigung, des Wertes der Atemfrequenz und dergleichen. Im Folgenden seien derartige, sich relativ langsam ändernde Daten als Meßwerte und derartige, sich relativ schnell ändernde Daten als Kurven bezeichnet.

Bei mindestens einem dieser medizinisch relevanten Daten handelt es sich dabei um ein sich relativ schnell änderndes Datum, also um eine Kurve. Weiterhin muß diese Kurve insofern eine Periodizität aufweisen, als sich deren grundsätzliche Muster in bestimmten Intervallen wiederholen, wie dies z.B. beim Elektrokardiogramm oder der arteriellen Pulskurve mit jedem Herzschlag oder bei der Kurve des Atemwegsdrucks mit jedem Atemzug der Fall ist.

Auf einer Anzeigeeinheit, die einem medizinischen System zugeordnet ist und die mit einer oder mehreren Meßwerterfassungsvorrichtungen in Verbindung steht, werden die genannten mindestens drei medizinisch relevanten Daten derart dargestellt, daß sich für den Benutzer des Systems ein scheinbar dreidimensionaler Eindruck der Darstellung ergibt. Die Darstellung dieser mindestens drei medizinisch relevanten Daten wird vorzugsweise in periodischen Intervallen erneuert, wobei die Intervalle von den Intervallen, welche die Periodizität der genannten Kurve bedingen, abgeleitet sein können.

Bei mehr als drei darzustellenden Daten können die vierte und weitere Dimensionen durch weitere Merkmale der dreidimensional dargestellten Kurve oder Fläche, wie Farbe, Graustufen, Strichstärke, Strichdichte, Helligkeit und ähnliches repräsentiert werden.

Der genannte dreidimensionale Eindruck entsteht durch Darstellung der genannten mindestens drei Daten in einem mindestens dreidimensionalen Koordinatensystem und die Transformation dieser Darstellung auf ein zweidimensionales Anzeigemittel gemäß einer vorher definierten Rechenvorschrift, welche in ihren Parametern vom Anwender veränderbar sein kann, sowie durch den dreidimensionalen Effekt unterstützende Merkmale, wie flächige Darstellung, Farbgebung, scheinbare Beleuchtung etc.

Die Erfindung wird nachfolgend anhand eines in der Zeichnung dargestellten Ausführungsbeispiels näher erläutert. In der Zeichnung zeigen:
- Fig. 1: Abschnitte von Kurven für Atemwegsdruck p, Fluß F und Volumen V, sowie die Schleifendiagramme V(p) und V(F) in einer bei bekannten Überwachungssystemen üblichen Art und Weise;
- Fig. 2: eine Darstellung der Daten der in Fig. 1 gezeigten Parameter in einer erfindungsgemäß komprimierten Darstellung;
- Fig. 3a: einen beispielhaften Bildschirminhalt eines Patientenüberwachungsmonitors gemäß dem bekannten Stand der Technik und
- Fig. 3b: einen beispielhaften Bildschirminhalt eines Patientenüberwachungsmonitors nach der Erfindung, auf dem die in Fig. 3a dargestellten Daten in erfindungsgemäß komprimierter Form dargestellt sind.

Die Erfindung wird im folgenden am Beispiel der Spirometrie, d.h. der Erfassung und Darstellung atemmechanischer Parameter erläutert.

Zur Überwachung kritisch kranker, beatmeter Patienten hat sich die Überwachung atemmechanischer Parameter als medizinisch sinnvoll erwiesen. Als Basis zur Überwachung dieser atemmechanischen Parameter werden in der Regel die folgenden Größen gemessen: Der Druck im Atemkreis und dessen Verlauf über der Zeit, der Gasfluß zum und vom Patienten und dessen Verlauf über der Zeit, sowie das dem Patienten zugeführte Volumen und dessen Verlauf über der Zeit. Die Messung von Gasfluß und Volumen ist redundant, da das Volumen das zeitliche Integral des Gasflusses ist; daher wird häufig nur der Fluß gemessen und aus diesem das Volumen durch Integration über der Zeit bestimmt.

Zur Messung der genannten Größen haben sich mehrere Verfahren etabliert, von denen eines der gängigsten im US-Patent 5,111,827 mit dem Titel "Respiratory Sampling Device" beschrieben ist, welches hier als Referenz genannt sei. Demgemäß wird in das Beatmungssystem ein Verbindungsstück eingebracht, welches eine Querschnittsverringerung des Beatmungsschlauches beinhaltet. Infolge des Gasflusses durch dieses Verbindungsstück stellt sich eine Druckdifferenz über der Querschnittsverringerung ein, deren Höhe ein Maß für die Flußgeschwindigkeit des durchströmenden Gases ist. Aus dieser Flußgeschwindigkeit läßt sich, wie oben beschrieben das Volumen des durchströmenden Gases ermitteln. Weiterhin läßt sich aus dem Vorzeichen der Druckdifferenz die Strömungsrichtung ermitteln. Die Messung des im Verbindungsstück herrschenden Drucks gegenüber dem atmosphärischen Druck liefert den Druck im Atemkreis. Es können noch zusätzliche Parameter zur Kompensation der Meßergebnisse gegenüber physikalischen Einflußgrößen, wie z.B. der Zusammensetzung, relativen Feuchte oder Temperatur des durchströmenden Gases herangezogen werden. Die genannten Größen Druck und Fluß werden von geeigneten Meßwertaufnehmern erfaßt, deren Ausgangswerte gegebenenfalls aufbereitet, anschließend digitalisiert, einer oder mehreren Verarbeitungseinheiten zugeführt und auf einer oder mehreren Anzeigemitteln dargestellt. Dieses Meßverfahren ist unter dem Begriff Spirometrie im medizinischen Alltag gebräuchlich und in einer Reihe kommerzieller Patientenüberwachungsmonitore und Beatmungs- und Anästhesiegeräte integriert.

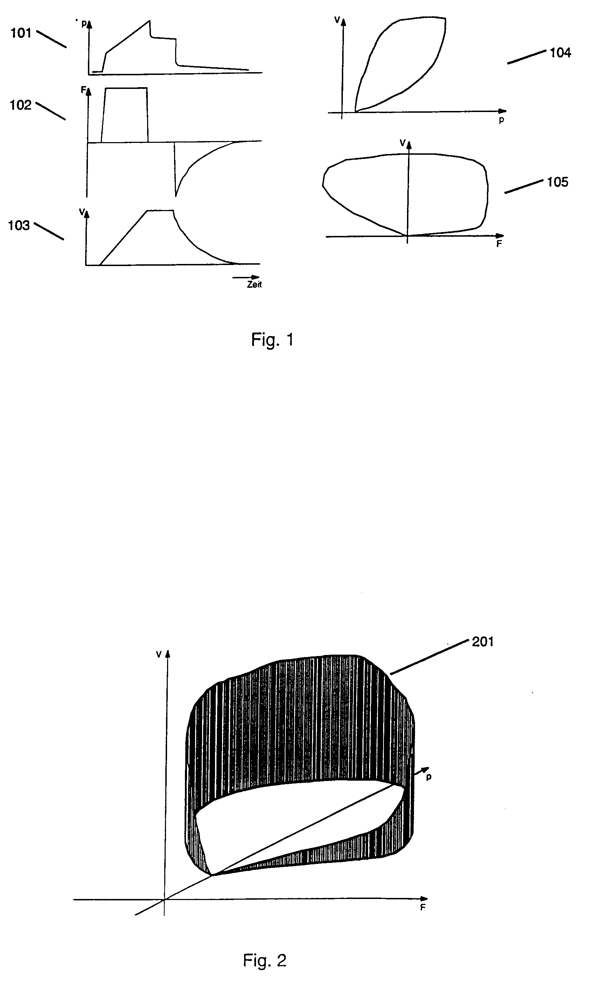

Als Darstellungsform auf Anzeigemitteln dieser genannten Geräte wird in der Regel die Kurvendarstellung der Größen Atemwegsdruck (Diagramm 101, Fig. 1), im folgenden mit Druck p bezeichnet und/oder Atemgasfluß (Diagramm 102, Fig. 1), im folgenden mit Fluß F bezeichnet und/oder Atemvolumen (Diagramm 103, Fig. 1), im folgenden mit Volumen V bezeichnet, verwendet. Zusätzlich zur Kurvendarstellung, d.h. der Zeitabhängigkeit der Parameter p, F, V, hat sich eine weitere Darstellungsform etabliert, welche die Größen Druck p und Volumen V, sowie Fluß F und Volumen V in jeweils einem zweidimensionalen Koordinatensystem als Abhängigkeiten V(p) und V(F) aufträgt. Da sich die wesentlichen Merkmale aller drei Kurven mit jedem Atemzug wiederholen, werden diese Darstellungsformen auch als Schleifendiagramme bezeichnet. Diese Schleifendiagramme sind ebenfalls in Fig. 1 als Diagramme 104 und 105 gezeigt.

Aus bestimmten charakteristischen Merkmalen dieser drei Größen können nun weiterhin verschiedene atemmechanische Kenngrößen ermittelt werden. Als Beispiele solcher atemmechanischer Kenngrößen seien genannt: Atemfrequenz, Spitzendruck, Plateaudruck, Mitteldruck,, PEEP (Positve End-Expiratory Pressure), Atemminutenvolumen, inspiratorisches und exspiratorisches Atemzugvolumen, inspiratorischer und exspiratorischer Spitzenfluß, Compliance, inspiratorische und exspiratorische Resistance, das Verhältnis Inspirationszeit zu Exspirationszeit, usw. Eine gute Erklärung dieser und weiterer atemmechanischer Kenngrößen und ihrer medizinischen Bedeutung ist beispielsweise in A. Gärtner, "Beatmungs- und Narkosetechniken", Verlag TÜV Rheinland, zu finden, das hierfür als Referenz genannt sei. Dort sind auch die typischen Kurvenverläufe für die Druck-, Fluß- und Volumenkurve, sowie typische Schleifendiagramme gezeigt. Weitere Erklärungen der atemmechanischen Grundlagen und deren Herleitung finden sich z.B. in Anaesthesist Vol. 42, S. 396-417, "Beatmungsgeräte in der Intensivmedizin" und in Anaesthesist Vol. 42, S. 813-832, "Moderne Beatmungsformen"; beide seien deshalb als Referenz genannt.

Aus den Kurvenverläufen 101 bis 103 und/oder den Formen der Schleifendiagramme 104, 105 kann eine entsprechend ausgebildete medizinische Fachkraft durch einfache Betrachtung der Form und charakteristischer Merkmale wichtige Erkenntnisse über den Zustand eines Patienten und seiner Atemmechanik ziehen. Als einfaches Beispiel sei hier das charakteristische Merkmal eines PEEP und dessen Erkennung anhand des Druck/Volumen-Schleifendiagramms (104) gezeigt.

Beim sogenannten PEEP (Positve End-Expiratory Pressure) handelt es sich um eine Erscheinung, bei der am Ende eines Atemzyklus der Atemwegsdruck nicht, wie normalerweise üblich, auf Null zurückgeht, sondern sich, z.B. aufgrund bestimmter Krankheitsformen oder bestimmter Beatmungsformen, gewollt oder ungewollt ein positiver Wert einstellt. Im Druck/Volumen-Schleifendiagramm 104 aus Fig. 1 ist dieser PEEP deutlich als Rechtsverschiebung der dargestellten Schleife gegenüber dem Koordinatenursprung zu erkennen. Diese Interpretationsmöglichkeiten der Kurvenverläufe in den Diagrammen 101 bis 103 und der Schleifen in den Diagrammen 104, 105 durch eine entsprechend ausgebildete medizinische Fachkraft machen es häufig überflüssig, die exakten Werte der atemmechanischen Kenngrößen zu betrachten, wenn deren ungefähre Werte durch eine, von einem entsprechend geübten Anwender vorgenommene Merkmalsextraktion, aus den Kurvenverläufen 101 bis 103 und/oder Schleifendiagrammin 104, 105 gewonnen werden können.

Wenn man sich den Inhalt der Anzeige 300 eines Patientenüberwachungsmonitors nach gängigem Stand der Technik betrachtet, wie beispielhaft in Fig. 3a. gezeigt, so besteht dieser aus einem oder mehreren Anzeigebereichen für Kurven 301 und/oder aus einem oder mehreren Anzeigebereichen für Meßwerte 302 und/oder aus einem oder mehreren Anzeigebereichen für Grafiken 303, sowie möglichen weiteren ein oder mehreren Anzeigebereichen für weitere Daten 304, bei denen es sich z.B. um Patienten-Identifikationsdaten handeln kann.

Diese jeweiligen Bereiche 301 bis 304 können, je nach Ausführungsform des betrachteten Patientenüberwachungsmonitors, mehr oder weniger frei innerhalb der Anzeige (300) plazierbar oder in ihrer Position fest zugeordnet sein.

In jedem Fall bedingt jedoch die Darstellung der atemmechanisch relevanten Daten einen erheblichen, nicht zu vernachlässigenden Teil der zur Verfügung stehenden Fläche eines Anzeigemittels. Dadurch entsteht die Situation, daß für andere Parameter, die medizinisch gleichfalls relevant sind, keine ausreichende Fläche mehr zur Verfügung steht. In der Praxis wird dieses Problem häufig dadurch gelöst, daß auf die Darstellung einiger der Parameter verzichtet wird, obwohl deren Überwachung und Darstellung medizinisch vorteilhaft wäre. Eine andere Lösung ist die Aufteilung der angezeigten Parameter auf mehrere Anzeigemittel, was jedoch aufgrund der damit verbundenen Zusatzkosten und des zusätzlichen Platzbedarfs für weitere Anzeigemittel nur gelegentlich praktiziert wird.

Um nun den für die Analyse der Atemmechanik eines Patienten benötigten Platzbedarf am Bildschirm einer Anzeigeeinheit drastisch zu verringern wird erfindungsgemäß eine gegenüber dem bisherigen Stand der Technik sehr stark komprimierte Methode der Darstellung gewählt. Dabei wird unter Bezugnahme auf Fig. 2 von einem dreidimensionalen Koordinatensystem ausgegangen, welches gemäß der bevorzugten Ausführungsform der Erfindung ein kartesisches Koordinatensystem ist, jedoch auch ein zylindrisches, kugelförmiges oder beliebig geformtes Koordinatensystem sein kann. Nunmehr wird, wie in Fig. 2 gezeigt, ausgehend von der beispielhaften Zuordnung der drei Raumachsen x (horizontal), y (nach hinten) und z (vertikal) zu den Größen Fluß F, Druck p und Volumen V jeder Punkt, der nach bisher gängiger Kurvendarstellung auf einem Anzeigemittel dargestellt wurde, als Punkt im dreidimensionalen Fluß-Druck-Volumen-Raum dargestellt.

Um den dreidimensionalen Eindruck der durch diese Mehrzahl von Punkten gebildeten Figur 201 zu erhöhen, wird von jedem dieser Mehrzahl der Punkte zum jeweils nächsten Punkt aus der Mehrzahl der Punkte eine Verbindungslinie gezogen. Um den dreidimensionalen Eindruck dieser genannten Figur 201 weiterbin zu erhöhen, kann von jedem dieser Mehrzahl von Punkten eine senkrechte Verbindungslinie zum Punkt mit den gleichen Koordinatenwerten für Fluß und Druck wie dem Ursprungspunkt dieser Linie, jedoch dem Wert für Volumen gleich Null, gezogen werden.

Gemäß der beispielhaften Ausführungsform der Erfindung wird diese genannte Figur 201 im Fluß-Druck-Volumen-Raum nach jedem vollendeten Atemzug erstellt und auf einem Anzeigemittel eines Patientenüberwachungsmonitors dargestellt. Dabei wird eine Transformation des dreidimensionalen Fluß-Druck-Volumen-Raums in die zweidimensionale x-y-Fläche des Anzeigemittels vorgenommen, indem Punkte, welche im Hintergrund liegen, von solchen im Vordergrund überdeckt werden.

Gemäß der beispielhaften Ausführungsform erstreckt sich die Flußachse in der Horizontalen, die Volumenachse in der Vertikalen und die Druckachse nach hinten, was in Fig. 2 durch einen Winkel von 30° zwischen Druck- und Flußachse dargestellt ist. Weiterhin erfolgt eine unterschiedliche Farbgebung der inneren und äußeren Flächen an der sich ausbildenden ringförmigen Figur 201, wobei die Farben vom Anwender wählbar sein können.

Gemäß einem weiteren Aspekt der vorliegenden Erfindung läßt sich die Änderungsgeschwindigkeit der Parameter Fluß und/oder Druck anhand der Strichdichte der oben genannten senkrechten Verbindungslinien ablesen, wobei eine geringe Strichdichte eine hohe Änderungsgeschwindigkeit und eine hohe Strichdichte eine geringe Änderungsgeschwindigkeit bedeutet. Denn die Meßwerterfassung der Parameter p, F, V erfolgt üblicherweise durch eine Abtastung in äquidistanten Zeitabständen.

Gemäß eines weiteren Aspekts der vorliegenden Erfindung können auch mehr als drei Größen in einer komprimierten drei- oder mehrdimensiomalen Darstellung gezeigt werden, wobei dann die vierte und weitere Größen durch Veränderung von Attributen einer dreidimensionalen Figur, wie z.B. Farbe, Graustufe, Strichstärke, Strichdichte, Helligkeit und dergleichen dargestellt werden.

Gemäß eines weiteren Aspekts der vorliegenden Erfindung läßt sich die Skalierung, also die Relation des realen Parameterwertes zur x-y-Koordinate eines Anzeigemittels, der Achsen Fluß und/oder Druck und/oder Volumen vom Anwender einstellen und/oder kann sich selbst an die Größe der einzelnen Parameter anpassen, so daß die sich ergebenden Figuren auf dem zur Verfügung stehenden Platz eines Anzeigemittels vollständig darstellbar sind.

Gemäß eines weiteren Aspekts der vorliegenden Erfindung läßt sich zusätzlich zu einer ersten Figur (201), welche dem jeweiligen Atemzug eines Patienten. entspricht, eine zweite Figur, bevorzugt in anderen Farben, innerhalb oder außerhalb des genannten Fluß-Druck-Volumen-Koordinatensystems der ersten Figur 201 darstellen, so daß ein einfacher visueller Vergleich der beiden dargestellten ersten und zweiten Figuren durch den Anwender möglich ist. Die genannte zweite Figur kann dabei durch Speicherung der Daten einer ersten Figur, zu einem früheren Zeitpunkt, entstanden sein.

Gemäß eines weiteren Aspekts der vorliegenden Erfindung läßt sich die der Transformation des dreidimensionalen Fluß-Druck-Volumen-Raumes in die zweidimensionale x-y-Fläche eines Anzeigemittels zugrunde liegende Rechenvorschrift vom Anwender beliebig in ihren Parametern verändern, so daß eine beliebige Ansicht der sich ergebenden Figur 201 möglich ist. Beispielsweise kann ein Drehen, Kippen oder Neigen sowie ein Vergrößern, Verkleinern oder Verzerren der Darstellung gemäß Fig. 2 erfolgen.

Für die beispielhafte Anwendung der komprimierten Darstellung von Daten aus einer Spirometrieanwendung erweist sich die beschriebene Zuordnung der drei Raumachsen als sinnvoll, da sich gut interpretierbare Figuren ergeben. Die Volumendarstellung in der Senkrechten kommt der medizinischen Denkweise entgegen, wonach gängige Beatmungs- und Anästhesiegeräte einen elastischen Balg umfassen, welcher sich auf- und abbewegt und damit ein Volumen einem Patienten zuführen. Die Darstellung des Flusses in der Horizontalen erweist sich als günstig, da der Fluß im Laufe eines Atemzyklus sein Vorzeichen umkehrt und sich dadurch rechts-links-symmetrische Figuren ergeben. Die Darstellung des Drucks nach hinten verstärkt die Räumlichkeit der sich ergebenden Figuren, da der Druck im Verlauf des Atemzyklus in der Regel von einem Wert nahe Null auf einen Spitzendruck ansteigt und anschließend wieder auf einen Wert nahe dem Ursprungsdruck abfällt.

Neben dem infolge der komprimierten Darstellung erheblich reduzierten Platzbedarf auf einem Anzeigemittel, ist ein weiterer Vorteil Methode der komprimierten Darstellung gemäß vorliegender Erfindung die schnelle Erfaßbarkeit und leichte Interpretierbarkeit dieser Darstellung durch entsprechend ausgebildetes medizinisches Fachpersonal. Als Beispiel sei die bereits weiter oben beschriebene Erkennung des charakteristischen Merkmals eines PEEP anhand der Methode der Darstellung gemäß vorliegender Erfindung erklärt. Wie in Fig. 2 beispielhaft gezeigt, ist die sich aus den einzelnen Meßwerten gemäß der beschiebenen Methode ergebende Figur 201 deutlich gegenüber der durch die Fluß- und Volumenachsen definierten Ebene nach hinten verschoben. In ähnlicher Weise wie beim PEEP lassen sich auch andere atemmechanische Kenngrößen visuell von einer entsprechend ausgebildeten medizinischen Fachkraft extrahieren. Diese Merkmalsextraktion durch medizinisches Fachpersonal aus Darstellungsformen, welche für Nichtfachleute im Gebiet der Medizin nur geringe Aussagekraft besitzen, ist in der Medizin in verschiedenen Fachbereichen, wie z.B. der Sonographie, der Radiologie oder der Kardiologie üblich.

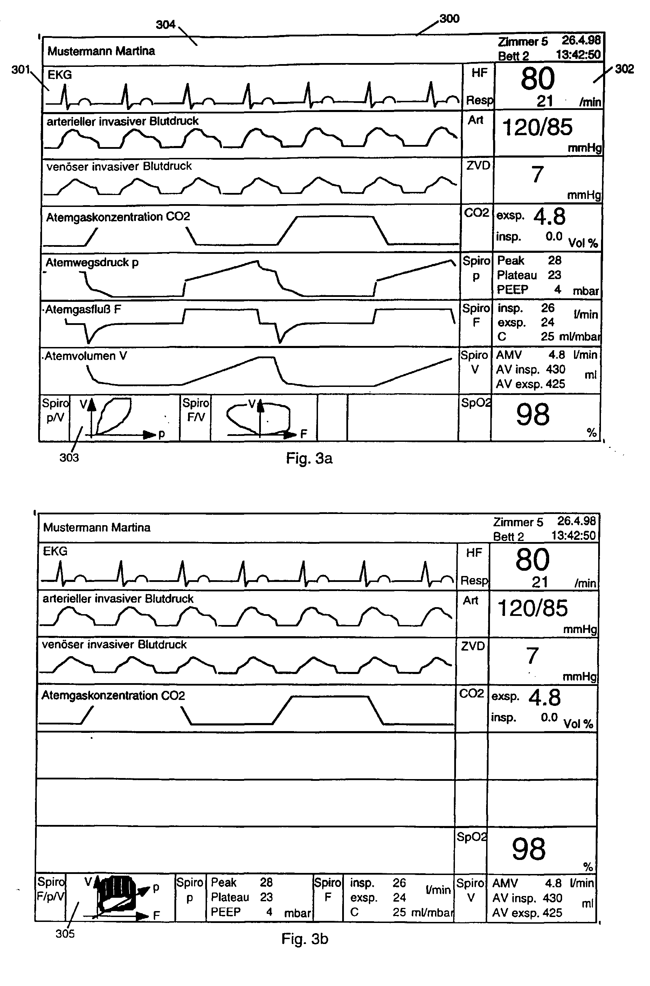

In Fig. 3b ist der Bildschirminhalt des beispielhaften Patientenüberwachungsmonitors aus Fig. 3a unter Ausnutzung der beschriebenen Methode zur komprimierten Darstellung medizinischer Daten gemäß vorliegender Erfindung gezeigt. Es ist offensichtlich, daß die Anwendung der dieser Erfindung zugrunde liegenden Methode eine erhebliche Platzersparnis auf einem Anzeigemittel im Vergleich zur bisher üblichen Darstellung gemäß Fig. 3a bedeutet. Dieser eingesparte Platz kann entweder für andere Parameter oder für eine übersichtlichere Bildschirmdarstellung oder zur Verkleinerung der Anzeigemittel oder für eine Kombination dieser Möglichkeiten verwendet werden. Die in Fig. 3b gezeigte dreidimensionale Figur 305 kann als Grafik verstanden werden, die je nach dem einem Patientenüberwachungsmonitor zugrunde liegenden Konzept mehr oder weniger frei innerhalb der Anzeige plazierbar oder in ihrer Position fest zugeordnet sein kann. Die in Fig. 3b gezeigte dreidimensionale Figur 305 kann zusätzlich zu der konventionellen Darstellung oder anstatt dieser auf einem Anzeigemittel dargestellt werden, wobei gemäß der Idee dieser Erfindung nach entsprechender Einarbeitung von Seiten des medizinischen Fachpersonals die ausschließliche Darstellung dieser dreidimensionalen Figuren, gegebenfalls ergänzt durch die Zahlendarstellung der weiter oben erwähnten atemmechanischen Kenngrößen, zur Beurteilung der Atemmechanik erfolgen sollte. Dies stellt einen wichtigen Schritt zur Reduzierung des anfangs erwähnten "mental overload" dar.

Es ist einsichtig, daß die Methode zur komprimierten Darstellung medizinischer Daten nicht auf das genannte Beispiel der Darstellung atemmechanisch relevanter Daten beschränkt ist, sondern sich vielmehr auf eine Vielzahl medizinischer Daten nutzbringend anwenden läßt. Es ist, um den Zweck der Erfindung zu erreichen, auch nicht zwingend erforderlich, daß es sich ausschließlich um genannte sich relativ schnell ändernde Daten, also gemäß obiger Definition um Kurven, handelt; vielmehr können sich relativ schnell ändernde Daten gemeinsam mit sich relativ langsam ändernden Daten gemäß der beschiebenen Methode komprimiert dargestellt werden. So ist es beispielsweise denkbar eine Kurve der Kohlendioxidkonzentration des Atemgases eines Patienten zusammen mit seinem Atemwegsdruck und seiner arteriellen Sauerstoffsättigung zu komprimieren. Bei genauer Betrachtung ergibt sich im medizinischen Bereich somit eine breite Anwendbarkeit der beschriebenen Methode.

## Patentansprüche

1. Verfahren zur gleichzeitigen komprimierten optischen Darstellung der Daten dreier oder mehrerer medizinisch relevanten Parameter,
a) bei dem die Daten der drei oder mehreren medizinisch relevanten Parameter in einem drei- oder mehrdimensionalen Koordinatensystem dargestellt werden,
b) bei dem das drei- oder mehrdimensionale Koordinatensystem zusammen mit den darin dargestellten Daten der medizinisch relevanten Parameter mit Hilfe einer vorgebenen Rechenvorschrift in ein zweidimensionales Koordinatensystem transformiert wird und
c) bei dem das Ergebnis dieser Transformation aufeiner Anzeigefläche eines Anzeigemittels angezeigt wird,
d) wobei die Daten mindestens eines der medizinisch relevanten Parameter einen in seinen wesentlichen Merkmalen zeitlich periodischen Verlauf aufweisen.

2. Verfahren nach Anspruch 1, **dadurch gekennzeichnet,** daß die Darstellung der drei oder mehreren Parameter auf dem Anzeigemittel regelmäßig oder unregelmäßig erneuert wird, vorzugsweise in zeitlichen Intervallen, die einer Periode eines der wenigstens einen periodischen Parameter entsprechen.

3. Verfahren nach Anspruch 2, **dadurch gekennzeichnet,** daß der Zeitpunkt der Erneuerung der Darstellung auf dem Anzeigemittel von einem oder mehreren der drei oder mehr medizinisch relevanten Daten beeinflußt wird.

4. Verfahren nach Anspruch 2, **dadurch gekennzeichnet,** daß der Zeitpunkt der Erneuerung der Darstellung auf dem Anzeigemittel von der Zeit beeinflußt wird.

5. Verfahren nach einem der vorhergehenden Ansprüche, **dadurch gekennzeichnet,** daß eine zusätzliche Koordinate des genannten drei- oder mehrdimensionalen Koordinatensystems die Zeit ist.

6. Verfahren nach einem der vorhergehenden Ansprüche, **dadurch gekennzeichnet,** daß mindestens einer der drei oder mehr medizinisch relevanten Parameter aus der Gruppe Atemwegsdruck (p(t)), Atemgasfluß (F(t)) oder Atemgasvolumen (V(t)) gewählt ist.

7. Verfahren nach einem der vorhergehenden Ansprüche, **dadurch gekennzeichnet,** daß das drei- oder mehrdimensionale Koordinatensystem ein kartesisches, zylindrisches oder Kugelkoordinatensystem ist.

8. Verfahren nach einem der vorhergehenden Ansprüche, **dadurch gekennzeichnet,** daß zur Verstärkung eines drei- oder mehrdimensionalen Eindrucks der Darstellung der drei oder mehreren Parameter vonjedem oder ausgewählten der die dargestellte Figur beschreibenden Punkten Verbindungslinien zu einer entsprechenden Anzahl von weiteren Punkten in dem drei- oder mehrdimensionalen Koordinatensystem dargestellt werden, vorzugsweise zu entsprechenden Punkten einer Projektion der Figur in eine Fläche, welche gekennzeichnet ist durch einen konstanten Wert zumindest eines Parameters.

9. Verfahren nach Anspruch 8, **dadurch gekennzeichnet,** daß zur Verstärkung eines drei- oder mehrdimensionalen Eindruck bestimmte Flächen des durch die von den Punkten gebildeten Figur und den Verbindungslinien beschriebenen drei- oder mehrdimensionalen Körpers in unterschiedlichen Farben und/oder mit unterschiedlichen Graustufen und/oder mit unterschiedlichen Strichstärken und/oder mit unterschiedlichen Strichdichten und/oder mit unterschiedlichen Helligkeiten dargestellt werden.

10. Verfahren nach einem der vorhergehenden Ansprüche, **dadurch gekennzeichnet,** daß bei vier oder mehr medizinisch relevanten Parametern drei der Parameter als dreidimensionale Figur dargestellt werden und der vierte oder weitere Parameter dargestellt werden durch mindestens ein Merkmal der Figur aus der Gruppe Farbe, Graustufe, Strichstärke, Strichdichte und Helligkeit der Darstellung.

11. Verfahren nach einem der vorhergehenden Ansprüche, **dadurch gekennzeichnet,** daß, zusätzlich zu den genannten drei oder mehr medizinisch relevanten Daten, die Zeit dargestellt ist und daß die Zeit mindestens ein Merkmal der Figur aus der Gruppe Farbe, Graustufe, Strichstärke, Strichdichte und Helligkeit der Darstellung beeinflußt.

12. Verfahren nach einem der vorhergehenden Ansprüche, **dadurch gekenn**zeichnet, daß zusätzlich zu der durch aktuelle Daten der medizinisch relevanten Parameter gebildeten Figur eine oder mehrere weitere Figuren innerhalb oder außerhalb des genannten drei- oder mehrdimensionalen Koordinatensystems dargestellt werden.

13. Verfahren nach Anspruch 12, **dadurch gekennzeichnet,** daß die ein oder mehreren weiteren Figuren nach der gleichen Rechenvorschrift transformiert werden, wie die aktuelle Figur.

14. Verfahren nach einem der Ansprüche 12 oder 13, **dadurch gekennzeichnet,** daß die ein oder mehreren weiteren Figuren mit anderen, den drei- oder mehrdimensionalen Eindruck verstärkenden Merkmalen als die aktuelle Figur dargestellt werden.

15. Verfahren nach einem der Ansprüche 12 oder 13, **dadurch gekennzeichnet,** daß die ein oder mehreren weiteren Figuren aus Daten der medizinisch relevanten Parameter abgeleitet werden, welche einem oder mehreren früheren Zeitintervallen einer dargestellten oder darstellbaren Figur oder Figuren entstammen.

16. Vorrichtung zur gleichzeitigen komprimierten optischen Darstellung der Daten dreier oder mehrerer medizinisch relevanten Parameter,
a) mit einer Auswerte- und Ansteuereinheit, welcher die Daten der medizinisch relevanten Parameter zugeführt sind, und
b) welche Anzeigemittel zur optischen Darstellung der Parameter ansteuert,
c) wobei die Daten mindestens eines der medizinisch relevanten Parameter einen in seinen wesentlichen Merkmalen zeitlich periodischen Verlaufaufweisen, und
d) wobei die Auswerte- und Ansteuereinheit so ausgebildet ist, daß die medizinisch relevanten Parameter nach dem Verfahren nach einem der vorhergehenden Ansprüche aufdem Anzeigemittel anzeigbar sind.

17. Vorrichtung nach Anspruch 16, **dadurch gekennzeichnet,** daß mit der Auswerte- und Steuereinheit Eingabemittel zur Eingabe von Daten zur Beeinflussung der Darstellung verbunden sind.

18. Vorrichtung nach Anspruch 17, **dadurch gekennzeichnet,** daß über die Eingabemittel Daten zur Beeinflussung der Rechenvorschrift zur Transformation des drei- oder mehrdimensionale Koordinatensystem zusammen mit den darin dargestellten Daten der medizinisch relevanten Parameter eingebbar sind.

19. Patientenüberwachungssystem mit einer Vorrichtung zur gleichzeitigen komprimierten optischen Darstellung der Daten dreier oder mehrerer medizinisch relevanten Parameter nach einem der Ansprüche 16 bis 18.

20. Beatmungs- oder Anästhesiegerät mit einer Vorrichtung zur gleichzeitigen komprimierten optischen Darstellung der Daten dreier oder mehrerer medizinisch relevanten Parameter nach einem der Ansprüche 16 bis 18.
